# EUROPEAN PATENT SPECIFICATION

(11) **EP 0 638 193 B1**
(45) Date of publication and mention of the grant of the patent: **02.09.1998**
(21) Application number: 93912039.0
(22) Date of filing: 21.04.1993
(51) Int. Cl.: G08B 21/00, A61B 5/0476

(54) **METHOD FOR SUPERVISING THE LEVEL OF ALERTNESS OF A PERSON**
VERFAHREN ZUR ÜBERWACHUNG DES WACHSAMKEITNIVEAUS EINER PERSON.
PROCEDE DE CONTROLE DU NIVEAU DE VIGILANCE D'UNE PERSONNE

(30) Priority: 21.04.1992 SE 9201255
(43) Date of publication of application: 15.02.1995
(73) Proprietor: PROMOTIONS S.A., Panama (PA)
(72) Inventor: RINDEGAARD, Bo, Panama 8 (PA)
(74) Representative: Waldinger, Ake
(86) International application number: SE9300350
(87) International publication number: WO9321615

(56) References cited:
- WO-A-87/03986
- US-A- 4 228 806
- US-A- 4 566 464

## Description

The present invention relates to a method for supervising the level of alertness of a person or individual, especially a vehicle driver, and indication of a non-permitted increase or decrease of the level of alertness in order to generate a warning signal at such an indication.

It is wellknown that the level of alertness of certain individuals might be too low with respect to the work they have to carry out. For certain professional groups, e.g. certain vehicle drivers, as air craft pilots,truck-drivers and locomotive drivers, it is very important that they have a very high level of alertness when they are driving their respective vehicles. It has become apparent, that especially air craft pilots after having been subjected to hard strain often are affected by rapid fall into a state of inactivity, which might be fatal. Similar phenomenons may affect air craft pilots when they are subjected to so called gravity attraction action during heavy accelerations. It is also known to locomotive drivers that monotony and loneliness during long nightly working periods creates need of sleep that moreover is amplified at the presence of infrasonic sound at the working place. A study has recently been performed on locomotive drivers wherein their EEG-signals were registered during nightly drivings along a specific route. Especially towards the morning hours it happened that they slumbered for up to 2-3 minutes. The registrations showed, that the drivers were running the red lights and missed traffic signs. Not until later they waked up and wondered what had happen. The tired or sleepy individual thinks that the body should give a last warning signal and then he intends to have a break. But no more warning signals appear, the sleepiness per se is an attempt by the brain to stop activity, and at last it is successful. Sleepiness is an underestimated cause of inability, and yet we know the consequences: Tiredness makes us careless. In a gas generating plant the routine readings were checked over a time period of 20 years. A significant increase of misreads were observed during the night shifts. Tiredness results in slow reactions. The worst ability was observed between 4 and 7 a.m. In this early time the one-car accidents are five times more than later in the day. Also for many other professional groups, e.g. nuclear power plant supervising personnel a too low level of alertness can result in fatal consequences. We know now for instance that the Tjernobyl meltdown occurred at 01.35 a.m. and was caused by a fault committed by individuals. Also the Three Mile Island accident started in the middle of the night at 4 a.m.

Also an overstimulation of e.g. car drivers or nuclear power plant personnel, caused by stress or other grounds, may cause individuals to work or function in an unreliable way.

It is priorly known that the brain activity of individuals can be studied by registration of an electro encephalogram, a so called EEG. The electric activity of the brain is measured and the corresponding signal is frequency analyzed. The frequency content of the signal is a measure of the brain activity of the individual.

The object of the invention is to provide a simple and uncomplicated and absolutely safe method of the kind initially mentioned. This has been achieved according to the invention by the method as specified in the appended claims.

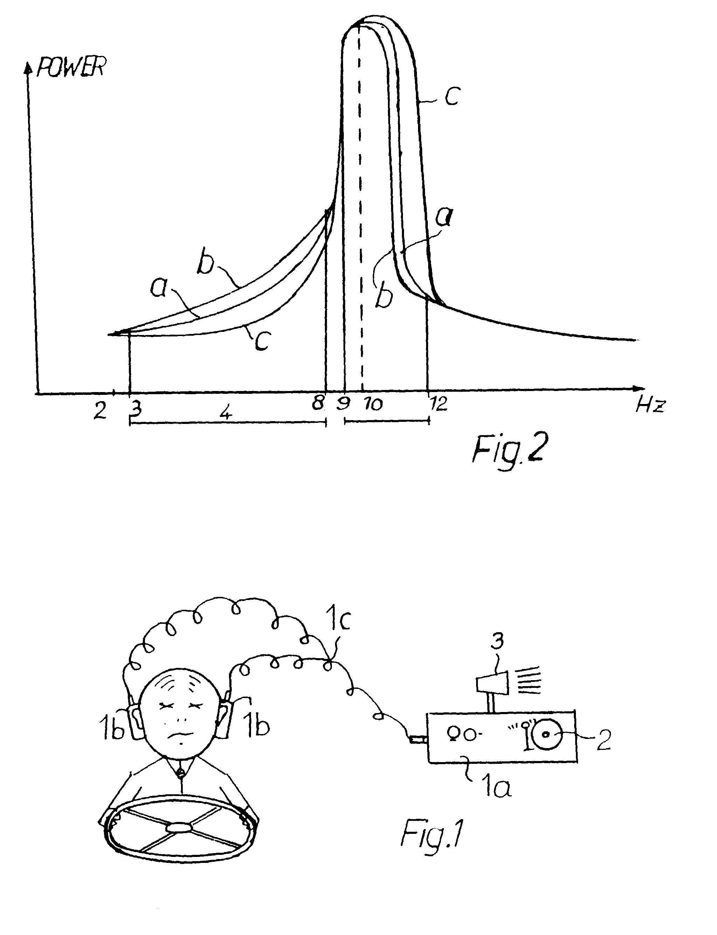

The method according to the invention is described more clearly in the following with reference to the appended drawing, in which figure 1 schematically discloses an example of an apparatus to be utilized in connection with the claimed method, and figure 2 discloses diagrams of the frequency distribution of the brain activity at three different characteristic conditions.

The apparatus shown in Fig. 1 comprises chiefly an electroencephalograph 1a for measuring of bioelectric currents of the brain of an individual and registration of EEG by means of biomedical electrodes 1b, which in a manner known per se are placed on a suitable place for easy measuring and availability on the head of the individual, e.g. just behind the ears. A flexible wire 1c connects the electrodes 1b to the apparatus 1a, but self-explanatory, if possible, a wireless transmission is preferred where possible. The embodiment shown is provided with two different alarm means 2,3, one of which 2 being intended to generate a characteristic signal indicating a non-permitted low level of alertness, and the other 3 a different signal indicating a non-permitted high alertness.

Fig. 2 discloses curves showing the effective output of the brain activity at various frequencies. A curve a shows the activity at normal alertness. It has become apparent that a curve b representing a non-permitted low alertness differs significantly from curve a showing a normal level of alertness, and, moreover, that a curve c showing a non-permitted high level of alertness differs significantly from curve b.

In the method according to the invention the most interesting part of the EEG is in the frequency range of 2-12 Hz. In the normal state the brain has a specific electric activity between 3 and 8 Hz and between 9 and 12 Hz, which ranges are marked 4 and 5, respectively, in Fig. 2. If the activity of the brain increases as compared to the normal state according to curve a in the frequency range 4 and at the same time decreases in the frequency range 5 this indicates that the level of alertness of the individual is going to decrease. Both the requirements of the increase and the decrease, respectively, of the activity in the two frequency ranges must be fulfilled if the level of alertness of the individual is to be accepted with certainty as decreasing. If the activity of the brain decreases in frequency range 4 as compared to the normal state according to curve a at the same time as it increases in frequency range 5 this indicates an overstimulation of the individual. Also in this case both the requirements of the decrease and increase, respectively, of the activity in the two frequency ranges must be fulfilled if the level of alertness of the individual is to be accepted with certainty as increasing up to overstimulation.

The invention as claimed in claims 1-3 and 5 means that a warning signal is released if the mutual change of the electric activity of the brain as compared to the normal state in the frequency ranges 3-8 Hz and 9-12 Hz exceeds certain predetermined critical relative values. The invention as claimed in claim 4 means, moreover, that a warning signal is released if the change per unit of time in the two frequency ranges exceeds certain predetermined critical limit values, which gives rise to an especially rapid response when sudden, rapid changes occur. This warning signals are meant for warning the individual in question and/or starting a protective or safety measure. The invention makes it possible to have a warning for non-permitted low as well as high level of activity of the individual, which means non-permitted low and high, respectively, level of alertness, which latter indicates that the individual is overstimulated or stressed.

In the method according to the invention the critical relative values and limit values are determined when the level of alertness becomes either non-permitted low or high. These relative values and limit values are varying for various professional groups depending on the risk they are running themselves or the risk they are exposing other peoples to, for example locomotive drivers, air craft pilots, security personnel and car drivers.

The alarm used can be of acoustic, visual or any other nature or a combination of two or more types of alarm.

## Claims

1. Method for supervising the level of alertness of a person or individual, especially a vehicle driver, and indication of non-permitted increase or decrease of the level of alertness in order to generate a warning signal at such an indication, **characterized** in that the electric activity of the brain is supervised by registering an electroencephalogram, which is continuously frequency analyzed, and in that an alarm (2,3) is arranged to be released at a predetermined simultaneous deviation of said registering in opposite directions from the normal awake state values within two specific frequency ranges, a lower range (4) and a higher range (5), where the deviations should amount to certain critical relative values.

2. Method according to claim 1, **characterized** in that a specific type of alarm (2) representing a non-permitted low level of alertness of the individual is used for being released if values representing an increase of the activity of the brain are registered in the lower frequency range (4) at the same time as values are registered representing a decrease of the activity of the brain in the higher frequency range (5), and the change as compared to the normal condition amounts to the predetermined, critical relative values.

3. Method according to claim 1 or 2, **characterized** in that a specific type of alarm (3) representing a non-permitted high level of alertness of the individual is used for being released if values representing a decrease of the activity of the brain are registered in the lower frequency range (4) at the same time as values are registered representing an increase of the activity of the brain in the higher frequency range (5), and the change as compared to the normal condition amounts to the predetermined, critical relative values.

4. Method according to any of the preceding claims, **characterized** in that the change relative to the normal condition includes a change per unit of time of the activity of the brain that exceeds stipulated, critical limit values.

5. Method according to any of the preceding claims, **characterized** in that the lower frequency range is set to 3-8 Hz and the higher one to 9-12 Hz.

## Patentansprüche

1. Verfahren zur Überwachung des Wachsamkeitniveaus einer Person oder eines Individuums, insbesondere eines Fahrzeugführers, und Anzeigen eines unzulässigen Anstiegs oder Abfalls des Wachsamkeitniveaus, um bei einem solchen Anzeigen ein Warnsignal zu erzeugen, **dadurch gekennzeichnet,** daß die elektrische Aktivität des Gehirns durch Erfassen eines Elektroenzephalogramms überwacht wird, das kontinuierlich frequenzanalysiert wird, und daß bei einer vorbestimmten, gleichzeitigen Abweichung der erfaßten Werte in zwei spezifischen Frequenzbereichen, einem niedrigeren Bereich (4) und einem höheren Bereich (5), in entgegengesetzten Richtungen von den Normalwerten des Wachzustandes ein Alarm (2, 3) ausgelöst wird, wenn die Abweichungen bestimmte kritische Relativwerte annehmen.

2. Verfahren nach Anspruch 1, **dadurch gekennzeichnet,** daß eine bestimmte Art von Alarm (2), der ein unzulässig niedriges Wachsamkeitniveau des Individuums anzeigt, ausgelöst wird, wenn einen Anstieg der Gehirntätigkeit anzeigende Werte im niedrigeren Frequenzbereich (4) und gleichzeitig einen Abfall der Gehirntätigkeit in dem höheren Frequenzbereich (5) anzeigende Werte erfaßt werden und die Veränderung im Vergleich zu dem Normalzustand die vorbestimmten, kritischen Relativwerte annimmt.

3. Verfahren nach Anspruch 1 oder 2, **dadurch gekennzeichnet,** daß eine bestimmte Art von Alarm (3), der ein unzulässig hohes Wachsamkeitniveau des Individuums anzeigt, ausgelöst wird, wenn einen Abfall der Gehirntätigkeit anzeigende Werte in dem niedrigeren Frequenzbereich (4) und gleichzeitig einen Anstieg der Gehirntätigkeit anzeigende Werte in dem höheren Frequenzbereich (5) erfaßt werden und die Veränderung im Vergleich zu dem Normalzustand die vorbestimmten, kritischen Relativwerte annimmt.

4. Verfahren nach einem der vorhergehenden Ansprüche, **dadurch gekennzeichnet,** daß die Veränderung im Vergleich zu dem Normalzustand eine Veränderung der Gehirntätigkeit pro Zeiteinheit einschließt, die festgesetzte, kritische Grenzwerte übersteigt.

5. Verfahren nach einem der vorhergehenden Ansprüche, **dadurch gekennzeichnet,** daß der niedrigere Frequenzbereich auf 3 - 8 Hz und der höhere Frequenzbereich auf 9 - 12 Hz eingestellt wird.

## Revendications

1. Procédé de supervision du niveau de vigilance d'une personne ou d'un individu, en particulier un conducteur de véhicule, et d'indication de l'augmentation ou de la diminution non permises du niveau de vigilance afin de générer un signal d'alarme pour une telle indication, caractérisé en ce que l'activité électrique du cerveau est supervisée par l'enregistrement d'un électroencéphalogramme, qui est analysé en fréquence en continu, et en ce qu'une alarme (2, 3) est prévue pour être déclenchée pour un écart simultané prédéterminé dudit enregistrement dans des directions opposées par rapport à des valeurs d'état éveillé normales à l'intérieur de deux plages de fréquence spécifiques, une plage inférieure (4) et une plage supérieure (5), où les écarts doivent se monter à certaines valeurs relatives critiques.

2. Procédé selon la revendication 1, caractérisé en ce qu'un type spécifique d'alarme (2) représentant un niveau bas non permis de la vigilance de l'individu est utilisé afin d'être déclenché si des valeurs représentant une augmentation de l'activité du cerveau sont enregistrées dans la plage de fréquence inférieure (4) en même temps que des valeurs sont enregistrées représentant une diminution de l'activité du cerveau dans la plage de fréquence supérieure (5), et la variation comparativement aux quantités de condition normale pour les valeurs relatives critiques prédéterminées.

3. Procédé selon la revendication 1 ou 2, caractérisé en ce qu'un type spécifique d'alarme (3) représentant un niveau élevé non permis de la vigilance de l'individu est utilisé afin d'être déclenché si des valeurs représentant une diminution de l'activité du cerveau sont enregistrées dans la plage de fréquence inférieure (4) en même temps que des valeurs sont enregistrées représentant une augmentation de l'activité du cerveau dans la plage de fréquence supérieure (5), et la variation comparativement aux quantités de condition normale pour les valeurs relatives critiques prédéterminées.

4. Procédé selon l'une quelconque des revendications précédentes, caractérisé en ce que la variation par rapport à la condition normale comprend une variation par unité de temps de l'activité du cerveau qui dépasse des valeurs limites critiques stipulées.

5. Procédé selon l'une quelconque des revendications précédentes, caractérisé en ce que la plage de fréquence inférieure est réglée à 3 à 8 Hz et la supérieure à 9 à 12 Hz.
